# FASCICULE DE BREVET EUROPEEN

(11) **EP 1 230 546 B1**
(45) Date de publication et mention de la délivrance du brevet: **08.10.2003**
(21) Numéro de dépôt: 00981433.6
(22) Date de dépôt: 17.11.2000
(51) Int. Cl.: G01N 33/50

(54) **PROCEDE D'EVALUATION DE LA GENOTOXICITE D'UN COMPOSE**
VERFAHREN ZUR BEURTEILUNG DER GENOTOXIZITÄT EINER VERBINDUNG
METHOD FOR ASSESSING GENOTOXICITY OF A COMPOUND

(30) Priorité: 19.11.1999 FR 9914603
(43) Date de publication de la demande: 14.08.2002
(73) Titulaire: INSTITUT NATIONAL DE LA SANTE ET DE LA RECHERCHE MEDICALE (INSERM), 75654 Paris Cédex 13 (FR); INSTITUT PASTEUR DE LILLE, 59019 Lille Cédex (FR)
(72) Inventeur: PALLARDY, Marc, F-92190 Meudon (FR); MARZIN, Daniel, F-59350 Saint André (FR); MEINTIERES, Sophie, F-59000 Lille (FR); BIOLA, Armelle, F-92130 Issy les Moulineaux (FR)
(74) Mandataire: Bernasconi, Jean
(86) Numéro de dépôt international: FR0003206
(87) Numéro de publication internationale: WO01036970

(56) Documents cités:
- US-A- 5 229 265
- L. H. BOISE ET AL.: "Introduction of the cell survival gene bcl-xl improves the viability of CTLL-2 cells without affecting their IL-2 proliferative response. Implications for the development of bioassays." JOURNAL OF IMMUNOLOGICAL METHODS., vol. 191, no. 2, 27 mai 1996 (1996-05-27), pages 143-148, XP002146056 ELSEVIER SCIENCE PUBLISHERS B.V.,AMSTERDAM., NL ISSN: 0022-1759
- H. E. BROOME ET AL.: "Apoptosis and Bcl-2 expression in cultured murine splenic T cells" IMMUNOLOGY., vol. 84, no. 3, mars 1995 (1995-03), pages 375-382, XP002146057 BLACKWELL SCIENTIFIC PUBLICATIONS., GB ISSN: 0019-2805
- M. MANDAL ET AL.: "Bcl-2 modulates telomerase activity" JOURNAL OF BIOLOGICAL CHEMISTRY., vol. 272, no. 22, 30 mai 1997 (1997-05-30), pages 14183-14187, XP002146058 AMERICAN SOCIETY OF BIOLOGICAL CHEMISTS, BALTIMORE, MD., US ISSN: 0021-9258
- I. FLORES ET AL.: "Diacylglycerol kinase inhibition prevents IL-2-induced G1 to S transition through a phosphatidylinositol-3 kinase-independent mechanism" JOURNAL OF IMMUNOLOGY., vol. 163, no. 2, 15 juillet 1999 (1999-07-15), pages 708-714, XP002146059 THE WILLIAMS AND WILKINS CO. BALTIMORE., US ISSN: 0022-1767
- S. KHARBANDA ET AL.: "Role for Bcl-xl as an inhibitor of cystolic cytochrome C accumulation in DNA damage-induced apoptosis" PROCEEDINGS OF THE NATIONAL ACADEMY OF SCIENCES OF USA., vol. 94, juin 1997 (1997-06), pages 6939-6942, XP002146060 NATIONAL ACADEMY OF SCIENCE. WASHINGTON., US ISSN: 0027-8424
- G. DENG ET AL.: "Suppression of apoptosis in a cytotoxic T-cell line by interleukin 2-mediated gene transcription and deregulated expression of the protooncogene bcl-2" PROCEEDINGS OF THE NATIONAL ACADEMY OF SCIENCES OF USA., vol. 90, mars 1993 (1993-03), pages 2189-2193, XP002146061 NATIONAL ACADEMY OF SCIENCE. WASHINGTON., US ISSN: 0027-8424 cité dans la demande

## Description

L'invention concerne un procédé d'évaluation de la génotoxicité d'un composé susceptible de représenter un risque pour l'homme ou l'animal.

A l'heure où se développe la chimie combinatoire permettant la synthèse d'innombrables nouveaux composés, les études toxicologiques à court terme prennent une place grandissante dans le cadre des premières étapes du développement de ces composés, susceptibles de présenter un intérêt dans des industries telles que les industries pharmaceutiques, agronomiques, alimentaires, cosmétiques, etc.

En effet, le développement d'un nouveau composé passe par des études indispensables avant la toute première exposition humaine et qui constituent les "pré-requis". Ce sont au moins des études de toxicité par administration unique et subaiguë et l'évaluation basale du potentiel mutagène et génotoxique.

La plupart des substances cancérogènes étant mutagènes et inversement, il est essentiel que la toxicologie génétique utilise des tests fiables permettant de conclure en tout sécurité quant aux effets potentiellement mutagènes d'un composé, via des mutations géniques, chromosomiques ou encore génomiques au niveau des cellules somatiques ou germinales.

Afin d'évaluer la génotoxicité des composés d'intérêt, l'industrie fait appel à des tests permettant de mettre en évidence un effet clastogène (cassure des chromosomes) ou aneugène (anomalie du fuseau).

Cependant, les auteurs de la présente invention ont montré que la mise en évidence d'un effet clastogène peut être perturbée par des composés non clastogènes mais induisant de l'apoptose avec présence d'événements nucléaires. Ces composés testés ressortent donc comme "faux-positifs", ou "exagérément-positifs" et sont écartés de toute application ultérieure.

Les auteurs de la présente invention ont mis au point un procédé d'évaluation de la génotoxité d'un composé qui permet de s'affranchir de ce problème des "faux-positifs", ou "exagérément-positifs".

L'invention a plus précisément pour objet un procédé d'évaluation *in vitro* de la génotoxicité d'un composé, dans lequel on met en contact ledit composé avec au moins une cellule ou type cellulaire surexprimant le protooncogène bcl2 et/ou une protéine antiapoptotique apparentée, et on observe les effets génotoxiques dudit composé sur ladite cellule.

L'invention a en d'autres termes pour objet l'utilisation de cellules surexprimant le protooncogène bcl2 et/ou une protéine antiapoptotique apparentée pour évaluer la génotoxicité d'un composé en s'affranchissant des effets liés à l'apoptose seule.

Le composé à tester peut être n'importe quel composé d'origine naturelle ou synthétique, que l'on désigne indifféremment par "composé", "produit" ou "substance". Il peut s'agir d'un mélange de plusieurs molécules identifiées ou non, comme par exemple un extrait d'origine animale ou végétale. Le composé à tester peut avoir un intérêt thérapeutique, ou être utile dans l'industrie chimique, agrochimique, alimentaire ou cosmétique notamment.

Selon un premier mode de réalisation de l'invention, on peut observer ces effets génotoxiques positifs par la formation de micronoyau(x).

Ce test du micronoyau, décrit notamment par Matsuoka et al, 1993 et Kirsch-Volders et al, 1997, est basé sur le principe suivant :
Lors de la mitose des cellules de mammifères, les fragments de chromosomes ou des chromosomes entiers n'ayant pas subi la ségrégation ne rejoignent pas le noyau principal pendant la télophase et peuvent être observés au microscope sous forme de micronoyaux séparés du noyau principal.
Les fragments d'ADN donnant naissance aux micronoyaux peuvent avoir pour origine soit des lésions de l'ADN (effets clastogènes ou aneugènes de composés génotoxiques), soit la coupure consécutive à l'apoptose (effets de composés pro-apoptotiques).

Selon un deuxième mode de réalisation de l'invention, on peut observer des effets génotoxiques positifs d'un composé par la présence d'anomalies de nombre et/ou de structure des chromosomes en métaphase.

Ce test d'analyse des métaphases a été décrit notamment par Evans et al, 1987.

Le principe de ce test est le suivant : les cellules sont traitées par le composé à tester, puis on recherche par étalement et coloration des cellules bloquées en métaphases (par un agent bloquant comme le colcémide), les anomalies chromosomiques (cassures, réarrangements...).

Ces deux modes de réalisation (test du micronoyau et test d'analyse en métaphase) peuvent être avantageusement mis en oeuvre de façon complémentaire.

Ils n'excluent pas d'autres modes de réalisation du procédé de l'invention, mettant en oeuvre des cellules surexprimant bcl2 et/ou une protéine antiapoptotique apparentée.

Par ailleurs, certains composés nécessitant une activation métabolique pour exercer leurs effets génotoxiques, il est possible d'ajouter au composé à tester un activateur ou système d'activation métabolique tel que du "S9 mix" contenant une fraction microsomale subcellulaire de foie de rats (Kirkland et al, 1989), dans le procédé de l'invention.

Conformément à la présente invention, le composé à tester est mis en contact avec des cellules surexprimant le protooncogène bcl2 et/ou une protéine antiapoptotique apparentée. La surexpression de bcl2, connu comme inhibiteur de l'apoptose, crée un déséquilibre entre inducteurs et inhibiteurs de l'apoptose de la famille bcl2/bax. La surexpression de la protéine bcl2 prévient ainsi les cellules de l'apoptose.

Par "protéine anti-apoptotique apparentée à bcl2", on entend toute protéine de la famille bcl2 ayant une activité anti-apoptotique, les caractéristiques de cette famille étant décrits par Biolo et al, 1999.

L'homologie entre les différents protéines de cette famille est concentrée au niveau de trois régions appelées BH1, BH2 et BH3 qui contrôlent leurs capacités de dimérisation avec d'autres membres de la même famille ainsi que leurs fonctions de régulation de l'apoptose. Tous les membres anti-apoptotiques contiennent de plus un domaine BH4 localisé près de leur extrémité N-terminale. Ces protéines possèdent en outre à leur extrémité C-terminale des acides aminés hydrophobes qui semblent importants pour leur ancrage dans les membranes intracellulaires.

Parmi les protéines anti-apoptotiques apparentées à bcl2, on peut citer de manière préférentielle bcl-XL, qui présente une très forte homologie avec bcl2 (Biolo et al, 1999 ; Chao et al, 1995).

Les cellules utilisées sont des cellules eucaryotes et de manière préférentielle, des cellules de mammifères. Selon un mode de réalisation préféré de l'invention, il s'agit de cellules CTLL-2. Les cellules CTLL-2, bien connues de l'homme du métier, proviennent d'une lignée continue de lymphocytes T cytotoxiques qui est un sous-clone dérivant de la souris C57bl/6. Cette lignée est disponible à l'American Type Culture Collection sous le numéro ATCC TIB-214 et a été décrite dans un certain nombre de publications (Gillis et al, 1977 ; Hu et al, 1997). D'autres types cellulaires peuvent également être utilisés, comme par exemple L5178Y, CHO, V79, fibroblastes ou cellules de lymphomes humains ou animaux, ou d'autres cellules eucaryotes. Une lignée CTLL-2 peut être transfectée par un plasmide contenant bcl2 ou une séquence apparentée, selon les méthodes standard de transfection et transformation connues de l'homme du métier. La préparation de cellules CTLL-2-bcl2 a notamment été décrite dans l'article de Deng et al, 1993.

Conformément à la présente invention, les cellules peuvent être transfectées de manière à surexprimer une protéine anti-apoptotique apparentée à bcl2, telle que bcl-XL, selon les méthodes standard à la portée de l'homme du métier qui connaît les séquences des gènes correspondants (Bolse et al, 1993).

Le procédé de l'invention est efficace pour la détection de faux-positifs dus au phénomène apoptotique. En outre, il permet de déceler les produits présentant un véritable pouvoir clastogène ou aneugène sans être inducteur d'apoptose.

Sa sensibilité et sa reproductibilité en font un procédé valable pour évaluer la génotoxicité de composés à grande échelle.

Les figures et exemples suivants illustrent l'invention sans en limite la portée.

### LEGENDE DES FIGURES :

Dans l'ensemble des figures, les étoiles représentées ont la signification suivante :
* : p< 0,05 contre le contrôle négatif
** : p < 0,01 contre le contrôle négatif

La figure 1A représente une analyse des métaphases de lymphocytes humains traités avec de la dexaméthasone pendant 44 heures.

La figure 1B représente la mesure de l'apoptose dans les lymphocytes humains traités par la dexaméthasone selon le procédé de détection Annexine-V-FITC.

La figure 2A représente une analyse des métaphases des cellules CTLL-2 (trait plein) et CTLL-2-bcl2 (trait pointillé) traitées avec de la dexaméthasone.

La figure 2B représente la mesure de l'apoptose dans les cellules CTLL-2 (colonnes hachurées) et CTLL-2-bcl2 (colonnes blanches) traitées avec de la dexaméthasone selon le procédé de détection Annexine-V-FITC.

La figure 3A représente le nombre de cellules micronucléées parmi les cellules CTLL-2 (trait plein) et CTLL-2-bcl2 (trait pointillé) traitées avec de la dexaméthasone.

La figure 3B représente la mesure de l'apoptose dans les cellules CTLL-2 (colonnes hachurées) et CTLL-2-bcl2 (colonnes blanches) traitées avec de la dexaméthasone selon le procédé de détection Annexine-V-FITC.

La figure 4A représente le nombre de cellules micronucléées parmi les lymphocytes humains traités avec de l'étoposide pendant 4 heures.

La figure 4B représente la mesure de l'apoptose dans les lymphocytes humains traités avec de l'étoposide selon le procédé de détection Annexine-V-FITC.

La figure 5A représente une analyse des métaphases de lymphocytes humains traités avec de l'étoposide pendant 4 heures.

La figure 5B représente la mesure de l'apoptose dans les lymphocytes humains traités avec de l'étoposide selon le procédé de détection Annexine-V-FITC.

La figure 6A représente le nombre de cellules micronucléées parmi les cellules CTLL-2 (trait plein) et CTLL-2-bcl2 (trait pointillé) traitées avec de l'étoposide.

La figure 6B représente la mesure de l'apoptose dans les cellules CTLL-2 (colonnes hachurées) et CTLL-2-bcl2 (colonnes blanches) traitées avec de l'étoposide selon le procédé de détection Annexine-V-FITC.

La figure 7A représente une analyse des métaphases des cellules CTLL-2 (trait plein) et CTLL-2-bcl2 (trait pointillé) traitées avec de l'étoposide.

La figure 7B représente la mesure de l'apoptose dans les cellules CTLL-2 (colonnes hachurées) et CTLL-2-bcl2 (colonnes blanches) traitées avec de l'étoposide selon le procédé de détection Annexine-V-FITC.

### EXEMPLES :

### Exemple I : Test du micronoyau dans les cellules CTLL-2 et CTLL-2-bcl2

### A - Systèmes cellulaires

### 1) Cellules lymphocytaires CTLL-2

La lignée murine CTLL-2 est un sous-clone de lymphocytes T cytotoxiques dérivant de la souris C57bl/6 dont la prolifération est dépendante de l'InterLeukine-2 (IL-2) recombinante humaine.

Les cellules sont cultivées dans un milieu C1 RPMI 1640 (Gibco BRL) complet, avec :
10 % de sérum de veau foetal décomplémenté 30 minutes à 56°C (Gibco BRL)
0,01 % de pyruvate de sodium à 20 mg/ml (Sigma)
2 mM de L-glutamine (Gibco BRL)
22 mM d'HEPES (Sigma)
100 Ul/ml de pénicilline (Gibco BRL)
0,1 mg/ml de streptomycine (Gibco BRL)
5.10-5 M de β-mercapto éthanol (Merck)
1 ng/ml d'IL-2

Les protocoles de congélation, décongélation et d'entretien des cellules à 37°C sont les suivants :
- *congélation des cellules CTLL-2 et CTLL-2 Bcl2*
   Les cellules sont centrifugées 5 minutes à 200 g, et le culot est ajusté à 3,5.10⁶ cellules pour 0,5 ml de sérum de veau foetal décomplémenté, puis 0,5 ml de la suspension de cellules est transféré dans un cryotube contenant 0,4 ml de sérum de veau foetal décomplémenté et 0,1 ml de DMSO (Merck; lot K2308267-651) à raison de 3,5.10⁶ cellules /ml.
   Les cellules sont congelées progressivement (-1°C par minute) et conservées en azote liquide à -196°C.
- *décongélation des cellules CTLL-2 et CTLL-2 Bcl2*
   Le cryotube est placé au bain-marie à 37°C.
   Le contenu est transféré dans 49 ml de milieu RPMI 1640 complet C1 puis dilué au 1/10^{éme} dans du milieu C1.
   Après 24 heures en étuve à 37°C sous atmosphère à 5% de CO₂ et 95% d'humidité, en flacon de 50 ml (Falcon, Becton Dickinson) la culture est centrifugée 5 minutes à 200 g et le culot est remis en suspension dans 50 ml de C1 et conditionné en flacon de 10 ml.
- *entretien des cellules CTLL-2 et CTLL-2 Bcl2*
   Les cellules CTLL-2 sont cultivées en milieu C1 qui est renouvelé deux fois par semaine. Les cellules dénombrées en cellules de Malassez sont diluées de telle façon qu'au passage suivant la densité n'excède pas 300 000 cellules/ml, sachant que le temps de doublement est de 12 heures.
   Les cultures sont placées en étuve à 37°C, sous atmosphère à 5% de CO₂ et 95 % d'humidité.

### 2) Cellules CTLL-2-bcl2

Les cellules CTLL-2-bcl2 sont issues de la transfection par électroporation de cellules CTLL-2 par le plasmide pSFFV-bcl2-néo : (plasmide fourni par le laboratoire du Dr Korsmeyer, Washington University, Saint Louis, Missouri, référence du plasmide : 3088) contenant un insert Eco-RI de 1,9 kb codant pour la protéine bcl2 humaine placée sous le contrôle de la région LTR du virus SV40 ainsi qu'un gène de résistance à l'ampicilline et à la géniticine (G418), (Renvoizé et al, 1997).

Le plasmide est linéarisé par l'enzyme de restriction Scal à raison de 1 unité par site de coupure et par µg de plasmide, à 37°C pendant 16 heures. L'enzyme est ensuite inactivée à 60°C pendant 15 minutes, et le plasmide est précipité dans de l'éthanol 100 %. La transfection des cellules avec le plasmide (10 µg de plasmide pour 10 millions de cellules) se fait par électroporation (Biorad, 250 V, 960 µF). Les cellules sont ensuite remises en culture dans du milieu complet pendant 48 heures, puis les transfectants stables sont sélectionnés en présence de 800 µg/ml de géniticine (Gibco) pendant au moins 15 jours, et ensuite par déprivation en IL-2 pendant 48 heures. Les cellules ainsi sélectionnées sont clonées par dilution dans des plaques 96 puits (Costar) à raison d'une cellule tous les deux puits.

La congélation, décongélation et entretien des cellules CTLL-2 bcl2 sont strictement les mêmes que pour les cellules CTLL-2.

### 3) Lymphocytes périphériques

Les échantillons de lymphocytes humains sont obtenus par isolement sur gradient de ficoll (Hypaque 1077; Sigma; lot 077H6024), après dilution du sang périphérique au 1/2 en RPMI 1640 (Gibco BRL; lot 3001360) contenant 0,08 %o d'héparine (Sanofi) pour un meilleur rendement.

Le sang a été recueilli par ponction veineuse par système vacutainer sur héparine-lithium (Becton Dickinson), en conditions stériles, chez un donneur volontaire sain, non fumeur, n'ayant reçu ni traitement médical récent ni radiation et n'ayant pas été affecté par aucune infection virale récente.

Les cellules sont cultivées à 37°C dans un milieu RPMI 1640 (Gibco BRL) complet C2 avec :
20% de sérum de veau foetal décomplémenté 30 minutes à 56°C (Gibco BRL)
22 mM de L-glutamine (Gibco BRL)
100 Ul/ml de pénicilline (Gibco BRL)
0,1 mg/ml de streptomycine (Gibco BRL)
100 Ul d'héparine
+2 mL de phytohémagglutinine A (Wellcome) pour 100 ml de milieu.

### B - Produits étudiés

### 1) Agents aneugènes

- *griséofulvine*
   La griséofulvine est un inhibiteur de la formation des microtubules. C'est un anti-mitotique : par son interaction avec des protéines associées aux microtubules polymérisés, elle perturbe le fuseau mitotique. La griséofulvine (Sigma; lot 85H07391) est mise en solution à 200 mM dans le DMSO (DiMéthylSulfOxyde), aliquotée et conservée à -20°C.
- *taxol*
   Le taxol est un agent anti-néoplasique dont l'efficacité clinique est prouvée. Ce composé présente des effets aneugènes et est un inducteur d'apoptose.
   Le taxol (Sigma; lot 126H1382) est mis en solution à 400 nM dans le DMSO, aliquoté et conservé à -20°C.
- *nocodazole*
   Le nocodazole affecte les microtubules et cause donc une aneuploïdie. D'autre part, comme le taxol, il provoque une phosphorylation de bcl2, un arrêt du cycle cellulaire en phase M et l'apoptose,
- *diethylstibestrol*
   Le diethylstibestrol (DES) est un oestrogène dont les propriétés pharmacologiques ont été mises à profit pour la prévention des avortements spontanés et qui continue à être utilisé chez l'homme dans la thérapie du cancer de la prostate. Ce composé augmente ou diminue la transcription de gènes régulés par les hormones.
   Il a été rapporté que le DES avait des actions cancérogènes chez l'homme, notamment après une administration pendant la grossesse par induction d'une aneuploïdie suite à son effet sur les centromères et les centrioles.
   Il s'avère que le DES a aussi une activité clastogène après activation métabolique.
- *diazépam*
   De même que le DES, le diazépam est un aneugène. Ce composé fait partie des benzodiazépines et a un effet anxyolytique et anti-convulsivant.

### 2) Agents clastogènes

- *mitomycine C*
   La mitomycine C est un antibiotique qui engendre des coupures simples brins de l'ADN et des cassures de chromosomes. Ce composé est un cancérogène possible chez l'homme.
   La mitomycine C (Ametycine; lot 440) est mise en solution à 500 µg/mL dans de l'eau pour préparations injectables, aliquotée et conservée à -20°C.
- *méthyl méthane sulfonate*
   Le Méthyl Méthane Sulfonate (MMS) est un agent alkylant monofonctionnel qui agit en déstabilisant l'ADN entraînant sa cassure. C'est un cancérogène possible chez l'homme.
   Le MMS (Aldrich; lot 030177) est mis en solution à 7 mM dans le RPMI, aliquoté et conservé à -20°C.
- *étoposide*
   La région centrale cyclique plane, flanquée d'un groupement phényl et d'un sucre fait de l'étoposide un inhibiteur non intercalant, spécifique et phase S-dépendant de la topoisomérase II.
   Cette enzyme ubiquitaire contrôle la topologie de l'ADN par la relaxation de l'ADN superenroulé, sa caténation et décaténation lors de la réplication, la transcription et la division cellulaire. Cette enzyme est essentielle pour la ségrégation des chromosomes, la recombinaison et la séparation des chromatides. L'étoposide bloque l'activité catalytique de la topoisomérase II en stabilisant le complexe ADN-topoisomérase II. Aussi, cette inhibition produit-elle des coupures de brins d'ADN, avec échanges de chromatides soeurs, aberrations chromosomiques et anomalies du nombre de chromosomes.
   C'est un inducteur d'apoptose : l'étoposide crée des cassures au niveau du matériel génétique et la protéine P53 arrête alors le cycle cellulaire en phase G1 pour permettre la transcription de gènes de réparation de l'ADN. Les cassures en nombre trop important dépassent le potentiel qu'ont les complexes de réparations à venir au secours du matériel génétique. P53 intervient alors pour déclencher le programme de mort cellulaire. On assiste à une apoptose P53-dépendante.
   Ces propriétés expliquent que l'étoposide soit à la fois un agent clastogène et un inducteur d'apoptose.
   L'étoposide (Sigma; lot 57H1159) est mis en solution à 20 mM dans le DMSO aliquoté et conservé à -20°C.
- *MNNG et MNU*
   La N-Méthyl-N'-Nitro-N-NitrosoGuanidine (MNNG) et la N-Méthyl-N-NitrosoUrée (MNU) sont deux agents alkylants induisant des lésions de l'ADN de type O6-méthylguanine, qui sont connues comme étant des lésions mutagènes. Ces composés produisent des lésions létales réparées par un autre mécanisme que celui impliquant l'alkyltransférase.
- *génistéine*
   La génistéine est une isoflavone abondante dans les produits dérivés du soja. Elle se comporte à la fois comme un agoniste et un antagoniste des récepteurs aux oestrogènes. Elle a aussi pour effet d'inhiber les Protéines à Tyrosines Kinases (PTK), les topoisomérases II, et d'induire la différenciation cellulaire et des événements d'oxydation. La génistéine provient de la société Sigma et est conservée dans du DMSO à -20°C.
- *CycloPhosphAmide*
   La CycloPhosphAmide (CPA) est métabolisée en un intermédiaire alkylant de l'ADN ce qui a pour effet d'interférer avec la synthèse d'ADN et la division cellulaire de façon phase-indépendante. Il est clairement établi que dans les cellules normales de la moelle osseuse ou de l'épithélium intestinal il y a blocage en phase G1/S pour réparer les lésions ou pour entrer en apoptose.

### 3) Inducteurs d'apoptose

- *déxaméthasone*
   La déxaméthasone est un glucocorticoïde qui induit l'apoptose selon deux modèles le modèle transcriptionnel nécessite une activation de gènes de mort cellulaire par le récepteur aux glucocorticoïdes, il n'y a donc pas d'action de la molécule au niveau de l'ADN. L'apoptose pourrait survenir selon un modèle transrépressionnel dans lequel il y aurait répression de facteurs nécessaires à la survie des cellules.
   La déxaméthasone (Sigma; lot 116H0427) est mise en solution à 150 mM dans le DMSO, aliquotée et conservée à -20°C.
- *gliotoxine*
   La gliotoxine est un métabolite fongique qui induit l'apoptose en créant une hyperphosphorylation dépendante des Protéine Kinase A, au niveau des résidus sérines des histones H3, rendant les cellules sensibles aux effets des nucléases.. La gliotoxine provient de la société Sigma et est conservée dans du DMSO à -20°C.
- *Méthional*
   Le méthional, qui est un métabolite de la méthionine, est un agent inducteur d'apoptose.

### C1 - Protocole du test du micronoyau sans activation métabolique

Les composés testés sont les suivants : griséofluvine, taxol, mitomycine C, MMS, étoposide, dexaméthasone, génistéine, gliotoxine, méthional, nocodazole et DES.

### - mise en oeuvre du test sur les cellules CTLL-2, CTLL-2 Bcl2

Dans des tubes de 15 ml, contenant chacun 5 ml de milieu RPMI 1640 complet additionné de 25 pg/ml d'IL-2, sont ajoutées 2.10⁶ cellules. On ajoute ensuite le produit à étudier à différentes concentrations selon une raison de 2. Parallèlement, un tube est traité par le solvant du produit à étudier (dans le cas du DMSO on ne dépassera pas 0.2 % en concentration finale) et un autre par un produit de référence positif (Mitomycine C à 75 ng/ml ; Boehringer; lot 1397592137) en solution aqueuse. Les tubes sont fermés à vis et vortexés doucement puis placés en position inclinée à l'étuve à 37°C sans agitation.

Les cellules CTLL-2 provenant d'une lignée continue en division permanente, sont toutes des cibles pour l'agent étudié et le traitement par la cytochalasine B (qui a pour effet de bloquer la cytokinèse) ne leur est donc pas indispensable. Il n'est pas nécessaire de limiter l'analyse aux cellules binucléées. De plus, la cytochalasine B est un agent dont l'effet risque d'interférer avec le potentiel clastogène des produits d'intérêt auquel les cellules sont très sensibles.

A la 15^{ème} heure après le début de l'incubation (1,5 cycles cellulaires), on pratique la récolte des lymphocytes. Les cellules sont lavées deux fois avec 5 ml de RPMI 1640 additionné de 2 % de sérum de veau foetal décomplémenté. Les cellules sont alors recueillies par centrifugation 5 minutes à 200 g, puis soumises 8 minutes à un choc hypotonique : (1 volume de RPMI 1640: 4 volumes d'eau pour préparations injectables + 2% de sérum de veau foetal décomplémenté). Après centrifugation, le surnageant est éliminé au maximum, et les cellules sont fixées par 10 ml de mélange fixateur de Carnoy II (3 volumes éthanol :1 volume acide acétique) pendant 10 minutes.

Après une nouvelle centrifugation, les cellules sont étalées sur lames et laissées sécher 24 heures à l'air libre, puis colorées pendant 10 minutes par le réactif de Giemsa dilué à 5 % dans l'eau.

Les cellules sont ensuite examinées microscopiquement au grossissement 1250 et on recherche les micronoyaux présents dans les cellules mononucléées.

### - mise en oeuvre du test sur les lymphocytes périphériques

Dans le cas des lymphocytes, la division est induite par la phytohémagglutinine A. Etant donné que seuls les lymphocytes T se divisent, il est indispensable d'ajouter de la cytochalasine B afin de n'examiner que les cellules binucléées ayant subi une mitose pour la recherche de micronoyaux,

Les cellules ayant subit 20 heures de préculture en milieu complet C2 additionné de phytohémagglutinine A (Murex Biotech; lot F067610) sont mises en présence du produit à étudier.

A la 44^{ème} heure après le début de l'incubation, les lymphocytes circulants sont lavés 2 fois par du milieu de culture à 10 % de svfd pour éliminer le produit, puis on ajoute du milieu complet contenant de la cytochalasine B (Sigma; lot 87H4054), à une concentration finale de 6 µg/ml.

A la 68^{ème} heure après le début de l'incubation, les lymphocytes sont récoltés et subissent le même traitement que ci-dessus. En revanche, les micronoyaux ne sont recherchés que sur les cellules binucléées.

### C2 - Protocole du test du micronoyau avec activation métabolique

Certains produits nécessitent une activation métabolique pour exercer leurs effets génotoxiques. *In vitro* cette activation se fait par l'utilisation du S9 Mix. Le S9 est une fraction microsomale subcellulaire de foie de rats induits par l'Aroclor. La composition du S9 mix (Kirkland et al, 1989) est la suivante :
2 ml S9 (préparé en laboratoire)
1 ml KCI à 150 g/l
1 ml Glucose-6-Phosphate à 180 g/l
1 ml NADP à 25 g/l

Cet essai est destiné à savoir si il y a une réponse en terme de génotoxicité ou d'apoptose suite à l'activation métabolique d'un composé par le S9 Mix.

Dans des tubes de 15 ml contenant chacun du milieu RPMI 1640 complet additionné de 25 pg/ml final d'interleukine 2 sont ajoutées 0,5.10⁶ cellules. Le volume final est de 5 ml.

On ajoute 0,25 ml de S9 Mix et le produit à étudier. Il s'agit dans cet exemple de cyclophosphamide (CPA).

Parallèlement, un tube est traité par le solvant du produit.

On incube au bain-marie à 37°C pendant 3 heures avec agitation à faible vitesse (B.M. GYROTORY G76: réglage sur 2,5, soit environ 80 cycles par minute).

Au bout de ce temps, on centrifuge et on élimine le surnageant.

On lave deux fois avec 5 ml de RPMI avec 10% de sérum de veau foetal décomplémenté.

On centrifuge et on élimine le surnageant jusqu'à environ la graduation 0,5 ml et on ajoute 4,5 ml de milieu RPMI complet additionné de 25 pg/ml d'interleukine 2.

On remet à l'étuve encore 15 heures.

La récolte se fait selon la même procédure que dans le test du micronoyau sans activation métabolique.

### D - Mesure de l'apoptose

### 1) Détection de l'apoptose par Annexin V-FITC (Euromedex)

Les cellules traítées en parallèle aux techniques de mutagenèse sont lavées en milieu de culture pour éliminer le produit et sont mises en suspension en tampon HEPES/NaOH (10 mM HEPES ; pH 7,4 ; 140 mM NaCI; 5 mM KCI ; 5 mM CaCl₂) à raison de 10⁶ cellules/ml.

100 µl de cette suspension sont mis en présence de 90 µl d'Annexin V-FITC (dilué en tampon HEPES à 10 µg/ml) pendant 15 minutes dans le noir à température du laboratoire. Enfin, on ajoute 10 µl d'iodure de propidium dilué en tampon HEPES à la concentration de 50 µg/ml.

L'analyse en cytométrie de flux est mise en oeuvre sur Epics Profile (Coulter Coultronic, Margency) : le niveau de fluorescence émise par les cellules marquées par l'Annexin-V est déterminé après son passage à travers un filtre à 525±10 nm et affiché après amplification logarithmique. La fluorescence émise par le iodure de propidium est mesurée à 600 nm.

### 2) Détection de l'apoptose par YOPRO-1 (Molecular Probes, Eugene, Oregon)

Les cellules traitées en parallèle aux techniques de mutagenèse sont lavées en milieu de culture pour éliminer le produit.

Le YOPRO-1 dilué (1V YOPRO-1:100V EPPI) est ajouté à raison de 1 µl/ 0,5.10⁶ cellules remises en suspension dans 0,5 ml de RPMI 1640.

L'analyse en cytométrie de flux est mise en oeuvre sur Epics Profile (Coulter Coultronic, Margency) : le niveau de fluorescence émise par les cellules marquées par le YOPRO-1 est déterminé après passage à travers un filtre à 525±10 nm et affiché après amplification logarithmique.

### E - Expression des résultats

Les résultats obtenus à l'issue de la recherche de micronoyaux et de l'induction de l'apoptose sont étudiés à l'aide du test du χ² par comparaison statistique des résultats à ceux obtenus avec le témoin solvant, ceci pour les études préliminaires.

Pour la recherche de micronoyaux en triplicate et l'étude de l'apoptose en duplicate, les auteurs de l'invention ont réalisé une analyse de variance à un facteur. En cas de significativité, le test de comparaison multiple de Dunnett a été réalisé.

Par l'analyse de variance de la régression, les auteurs de l'invention ont vérifié qu'il existe une corrélation significative entre le nombre de micronoyaux observés ou le pourcentage de cellules apoptotiques et la concentration en produit.

### F - Résultats

### 1) La surexpression de la protéine bcl2 élimine les faux positifs par apoptose

Les auteurs de l'invention ont montré qu'il existe une véritable corrélation entre les résultats obtenus sur les lignées murines CTLL-2 et CTLL-2-bcl2 en terme d'induction de micronoyaux dans le cas de cellules traitées par un agent aneugène strict (ariséofulvine) ou des agents clastogènes non reconnus comme étant inducteurs d'apoptose (mitomycine C et MMS). Quelle que soit la lignée, le phénomène apoptotique n'est pas déclenché tandis que les fréquences de cellules micronucléées augmentent significativement avec la dose. De surcroît, l'importance des manifestations clastogènes et aneugènes sont comparables dans les deux lignées.

Par ailleurs, afin de prouver que l'effet apoptotique se surajoute à l'effet génotoxique, les lignées ont été traitées par des agents aneugènes ou clastogènes qui sont également inducteurs d'apoptose: respectivement le taxol et l'étoposide. On montre effectivement que le modèle CTLL-2-bcl2 permet la distinction entre les effets dus à l'apoptose de ceux dus à la clastogenèse ou à l'aneugenèse. Puisque les cellules transfectées par le gène codant pour la protéine bcl2 ont la propriété de ne pas être sensibles aux agents apoptotiques en raison du déséquilibre artificiellement créé entre les membres de la famille bcl2, la concentration importante de la protéine est toujours supérieure à celle des protéines inductrices d'apoptose.

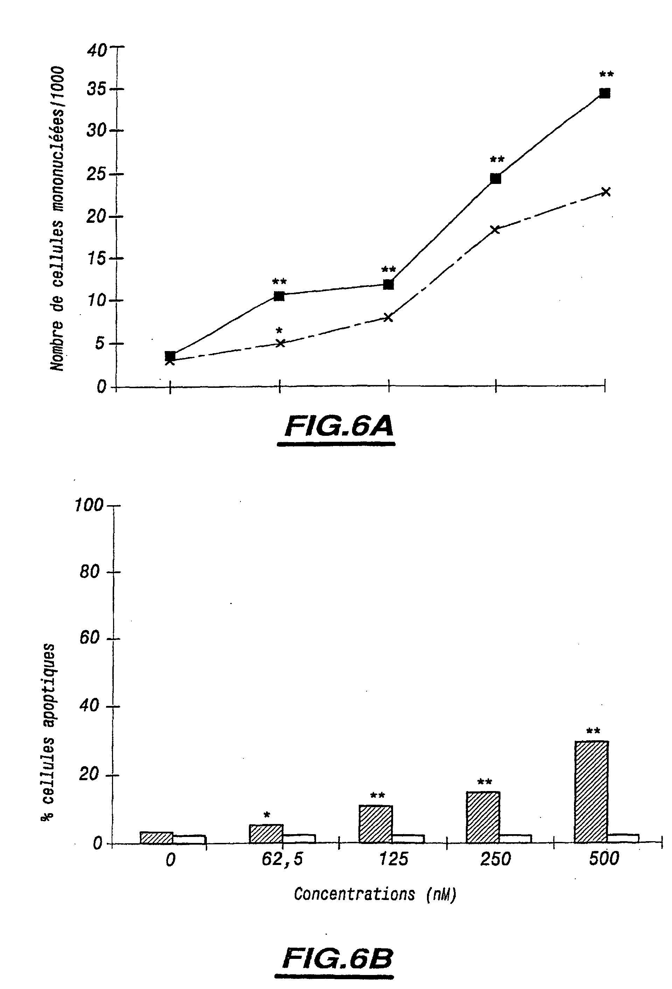

Bien qu'agissant par des mécanismes différents d'induction de l'apoptose, les deux agents engendrent des phénomènes comparables : tous deux induisent l'apoptose ainsi que la formation de micronoyaux dans les cellules CTLL-2. En revanche, dans les cellules CTLL-2-bcl2, la formation de micronoyaux est beaucoup plus faible. La différence d'évolution du nombre de micronoyaux entre la lignée CTLL-2 et la lignée CTLL-2-bcl2 apparaît déjà à la première concentration de taxol (25 nM). De même, elle a lieu à la première dose d'étoposide (62.5 nM; figures 6A et 6B).

A l'inverse, la lignée transfectée par le gène codant pour la protéine bcl2 inhibitrice d'apoptose, témoigne de l'effet génotoxique des agents étudiés en éliminant la composante apoptotique. Ainsi l'observation de l'évolution du nombre de micronoyaux de la figure 6A qui présentent des pentes différentes dans les lignées CTLL-2 et CTLL-2-bcl2 montre qu'une partie de l'induction des micronoyaux par l'étoposide est due au phénomène apoptotique et qu'une partie est due à l'effet clastogène propre du composé.

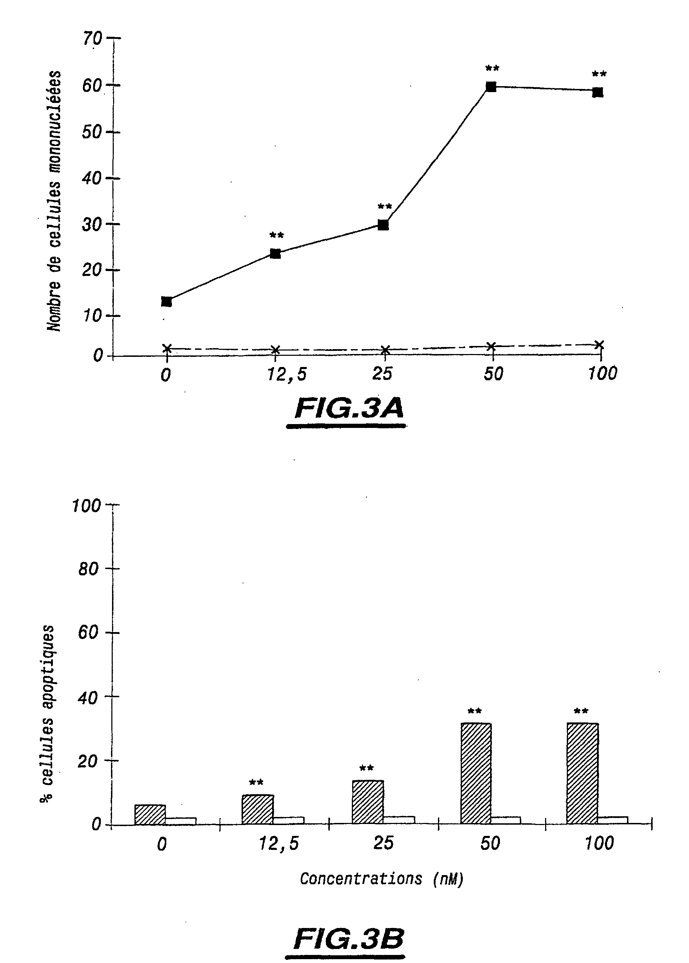

Les auteurs de l'invention ont induit la formation de micronoyaux et une apoptose seulement dans les cellules CTLL-2 à des doses entre 12,5 nM et 100 nM de déxaméthasone (figures 3A et 3B).

Dans ce modèle, le pourcentage de cellules micronucléées, qui était de 3 %₀ dans le contrôle négatif, a augmenté pour donner un maximum de 60 %₀ à partir de 50 nM de déxaméthasone.

De tels résultats permettraient de conclure que la déxaméthasone avait des propriétés mutagènes, mais le nombre de cellules avec des micronoyaux augmentait de manière similaire avec l'augmentation du pourcentage des cellules apoptotiques. Ce dernier a augmenté de 6 % (dans le contrôle négatif) à 30,5 % selon les méthodes avec Annexine-V-FITC et YOPRO-1 et un maximum a également été observé à partir de 50 nM de dexaméthasone.

Quelles que soient les concentrations en déxaméthasone, il n'a pu être observé ni augmentation *de* cellules apoptotiques ni augmentation de cellules micronucléés dans la souche CTLL-2-bcl2 suggérant encore que l'apoptose est responsable des résultats faussement positifs.

Les travaux sur la déxaméthasone, inducteur reconnu d'apoptose dans les cellules lymphocytaires, permettent donc de mettre en évidence l'intérêt qu'a le modèle CTLL-2-bcl2 à distinguer un produit génotoxique d'un produit apoptotique. Il ne laisse paraître aucune induction d'apoptose ni aucune formation de micronoyaux alors que la lignée CTLL-2 non transfectée a subi le phénomène apoptotique, qui s'accompagne d'une élévation du taux de cellules aberrantes. Ceci montre par la même que les micronoyaux ont pour origine les fragments d'ADN formés par des endonucléases activées lors du processus d'apoptose. Ces fragments issus de l'apoptose peuvent être mis en évidence par une image caractéristique en "échelle" après électrophorèse en gel d'agarose.

### 2) Le modèle CTLL-2/CTLL-2-bcl2 détecte les produits génotoxiques

Dans le test *in vitro* du micronoyau, un produit est classé comme génotoxique s'il provoque une augmentation statistiquement significative et dose-dépendante par rapport au témoin négatif de la fréquence de micronoyaux.

A l'issue des traitements par les clastogènes et aneugènes purs (mitomycine C, MMS, griséofulvine), on a pu noter dans chacune des deux lignées cellulaires non seulement une augmentation statistiquement significative de la fréquence d'aberrations, mais aussi une augmentation dose-dépendante : les critères permettant de classer un produit dans la catégorie des produits génotoxiques sont donc remplis. De plus les deux lignées ont la même sensibilité aux produits avec lesquels elles ont été mises en contact puisque pour une concentration donnée d'un produit testé, le nombre de cellules micronucléées est comparable dans les deux lignées, sans qu'il n'y ait d'apoptose visible.

Les critères de positivité sont également remplis dans l'étude des produits clastogènes ou aneugènes et à la fois inducteurs d'apoptose (étoposide et taxol) dans les lignées CTLL-2 et CTLL-2-bcl2. Toutefois, cette dernière lignée a en plus la propriété de ne mettre en évidence que le rôle aneugène et clastogène de ces deux produits: en effet, aussi bien avec l'étoposide (clastogène) qu'avec le taxol (aneugène) on note une augmentation de l'incidence des micronoyaux en dehors de tout évènement apoptotique. Dans le cas de l'étoposide, on observe une augmentation de la fréquence des micronoyaux dans les cellules CTLL-2-bcl2 de 12.5 à 100 nM qui n'entrent pas en apoptose. Avec le taxol, on observe une augmentation du nombre de micronoyaux à 25 nM sur cellules CTLL-2 et à 50 nM sur cellules CTLL-2-bcl2 alors que ces doses ne provoquent pas d'apoptose. Ces constatations montrent que la lignée CTLL-2-bcl2 est un bon modèle non seulement pour détecter les faux positifs dus à l'apoptose mais qu'en plus elle permet d'attribuer son rôle génotoxique à un composé.

De plus lors d'une étude sur un inducteur pur d'apoptose comme la déxaméthasone, on peut mettre en évidence le rôle de l'apoptose comme source de faux positifs puisque ses effets sur la lignée CTLL-2 sont caractéristiques d'un produit génotoxique, mais en aucun cas on ne peut lui attribuer un tel potentiel dans la lignée transfectée par le gène codant pour l'inhibiteur d'apoptose bcl2.

### 3) Les lignées CTLL-2/CTLL-2-bcl2 présentent une bonne sensibilité face aux produits étudiés

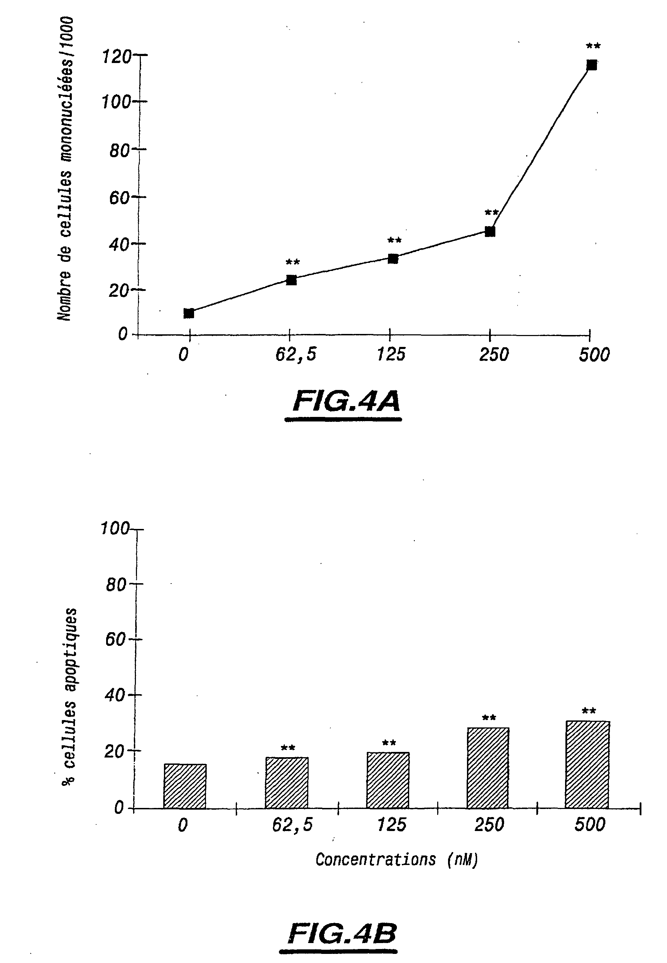

La qualité du modèle mis au point est confirmée par ailleurs par sa bonne sensibilité. On constate que pour chacun des produits testés les réponses sont comparables entre les essais menés en triplicate et ceux réalisés en screening et ceux notés avec les lymphocytes humains en culture (cf les figures 4A et 4B sur les lymphocytes humains en comparaison avec les figures 6A et 6B sur CTLL-2 et CTLL-2-bcl2). Par ailleurs on observe que les résultats obtenus avec les vrais génotoxiques (mitomycine C, MMS, griséofulvine) sont les mêmes dans les deux lignées, ce qui signifie que la surexpression de bcl2 ne modifie pas la sensibilité des cellules à l'effet aneugène ou clastogène d'un produit.

Enfin la reproductibilité entre résultats obtenus en criblage et résultats obtenus dans les essais en triplicate et la répétabilité des essais en triplicate, avec de faibles écarts-types, permettent de conclure à la bonne qualité d'interprétation des résultats.

Par l'étude de la gliotoxine ou du méthional, les auteurs de l'invention ont, comme dans le cas de la déxaméthasone, pu mettre en évidence le fait que le modèle CTLL-2/ CTLL-2-bcl2 permet de conclure que les produits n'induisant que de l'apoptose étaient responsables de la formation de micronoyaux, amenant à une conclusion faussement positive en terme de génotoxicité.

Comme dans le cas de l'étude avec le déxaméthasone, dans les cellules CTLL-2 traitées avec du méthional à des doses allant de 60,8 µM à 150 µM, il a été observé dans le même temps l'induction d'une apoptose et la formation de micronoyaux. Le niveau de cellules apoptotiques a atteint 24 % à la dose la plus forte contre 6,5 % dans le contrôle tandis que le nombre de cellules micronucléées augmentait jusqu'à 98 %₀, contre 11 %₀ dans le contrôle. Dans la détermination de cellules micronucléées comme dans l'induction de l'apoptose, les résultats étaient statistiquement significatifs (p=0,01), dès la première dose de méthional (60,8 µM). Quelle que soit la concentration en méthional, ni l'apoptose ni les micronoyaux ne sont apparus dans les cellules transfectées par bcl2. Dans ce cas également, les micronoyaux induits dans les cellules CTLL-2 étaient dus à l'apoptose.

La gliotoxine a également été étudiée à des doses allant de 25 nM à 200 nM dans le modèle développé. Tandis que l'apoptose était maintenue à un taux inférieur dans les cellules CTLL-2-bcl2, de même que le nombre de cellules micronucléées dans les cellules non transfectées, le niveau d'apoptose a augmenté de 7,5 % à 45 % à la dose la plus forte et le nombre de cellules aberrantes est monté de 5 %₀ à 20 %₀. Dans le test du micronoyau et dans le test d'apoptose, les résultats sont différents du contrôle (p=0,01) à partir de 100 nM.

### - Résultats complémentaires

Le nocodazole, la génistéine, la camptothécine, la bréfeldine A, l'anisomycine C, le curcumin, la quinacrine, la thapsigaraine induisent l'apoptose par des mécanismes différents dans la lignée CTLL-2.

Comme dans le cas de l'étoposide, on observe une réponse en terme de mutagenèse exagérée dans la lignée CTLL-2 par l'apparition de l'apoptose aux fortes concentrations de produits tandis que l'amplitude du nombre de cellules micronucléées est moindre dans la lignée CTLL-2-bcl2.

Le modèle CTLL-2/ CTLL-2 Bcl2 permet de différencier l'effet apoptotique de l'effet mutagène lors de la lecture des résultats du test du micronoyau sur CTLL-2.

La MNU, le diazepam et l'éthylméthanesulfonate (EMS) entraînent une réponse positive dans le test du micronoyau sur CTLL-2 et CTLL-2-bcl2 avec une même amplitude dans chacune des deux lignées sans induire d'apoptose, confirmant le fait que ce modèle permet de détecter des produits possédant des pouvoirs génotoxiques, que les produits soient ou non inducteurs d'apoptose.

L'actinomycine D et la staurosporine, comme dans le cas de la dexaméthasone, par exemple, induisent une réponse positive en terme de clastogenèse dans les cellules CTLL-2, corrélée à l'induction de l'apoptose. Dans les cellules CTLL-2-bcl2, ni apoptose ni génotoxicité ne sont observées, démontrant l'efficacité du modèle à éliminer l'interférence due à l'apoptose dans le test du micronoyau *in vitro*.

### - Résultats avec l'activation métabolique :

Après activation métabolique, la cyclophosphamide (CPA), le benzo[a]pyrène et le 7,12-diméthylbenz[a]anthracène (DMBA) induisent la formation de micronoyaux sur les cellules CTLL-2 et CTLL-2-bcl2, ainsi qu'une apoptose sur les CTLL-2 uniquement, montrant que ces cellules sont aptes à donner une réponse en terme de mutagénèse et d'apoptose suite à leur traitement par un mutagène direct.

### Exemple II : Test d'analyses de métaphases dans les cellules CTLL-2 et CTLL-2-bcl2

### A - Matériels

Les systèmes cellulaires sont les mêmes que dans l'exemple I (test du micronoyau).

### B - Principe

Les cellules murines sont traitées par le produit à étudier, puis on recherche par étalement et coloration des cellules bloquées en métaphases par la colcémide les anomalies de nombre et de structure chromosomiques.

L'annexine-V-FITC est la technique choisie pour déterminer le pourcentage de cellules en apoptose.

Les concentrations choisies correspondent aux doses utilisées dans le test *in vitro* du micronoyau dans ces cellules.

### C - Protocole

Dans des tubes de 15 ml contenant chacun du milieu RPMI 1640 complet sont ajoutées 1.10⁶ cellules.

On ajoute ensuite le produit à étudier à différentes concentrations, le volume final devant être de 10 ml.

Parallèlement, un tube est traité par le solvant du produit (dans le cas du DMSO, on ne dépasse pas 0,2 % en concentration finale) et deux autres par un produit clastogène de référence (MMS à 200 µM) et par un inducteur d'apoptose de référence (la dexaméthasone à 150 nM).

Les tubes sont fermés à vis et vortexés doucement puis placés en position inclinée à l'étuve sans agitation à 37°C.

A la 13^{ème} heure de traitement, la colcémide est ajoutée à la concentration finale de 100 ng/ml.

A la 15^{ème} heure de traitement, les cellules sont recueillies par centrifugation 5 minutes à 200 g et soumises 5 minutes à un choc hypotonique (KCI 75 mM).

Après centrifugation, le surnageant est éliminé au maximum et les cellules sont fixées par 10 ml d'un mélange fixateur de Carnoy (3V méthanol : 1V acide acétique) pendant 10 minutes.

Après une nouvelle centrifugation, les cellules sont étalées sur lames et laissées sécher 24 heures à l'air libre, puis colorées pendant 10 minutes par le réactif de Giemsa dilué à 4 % dans l'eau.

Les lames sont examinées au microscope et on recherche les aberrations chromosomiques (cassures, réarrangements, ...).

### D - Expression des résultats

Les résultats obtenus à l'issue des analyses de métaphases sont étudiés à l'aide de test t de Student. Bien que les écarts-types ne permettent pas de démontrer que la distribution est normale, le test peut être utilisé étant donnée la taille de l'échantillon. L'ensemble des aberrations numériques et l'ensemble du nombre de cellules avec ou sans gaps sont traités sur le plan statistique à l'aide du test de χ² ainsi que le nombre de cellules apoptotiques.

En premier lieu, les trois inducteurs purs d'apoptose reconnus, déxaméthasone, gliotoxine, méthional, ont été testés dans le test d'analyses de métaphases mis au point dans les cellules CTLL-2 et CTLL-2-bcl2 dans le but de voir si la fragmentation qui a lieu lors du phénomène apoptotique pouvait donner lieu à l'observation de cassures de chromosomes dans les cellules traitées arrêtées à la métaphase.

Puis, il a été recherché si la transfection de l'inhibiteur d'apoptose permettait d'observer une fréquence d'aberrations moindre quand les cellules sont traitées par un agent à la fois clastogène et inducteur d'apoptose comme l'étoposide.

### E - Résultats

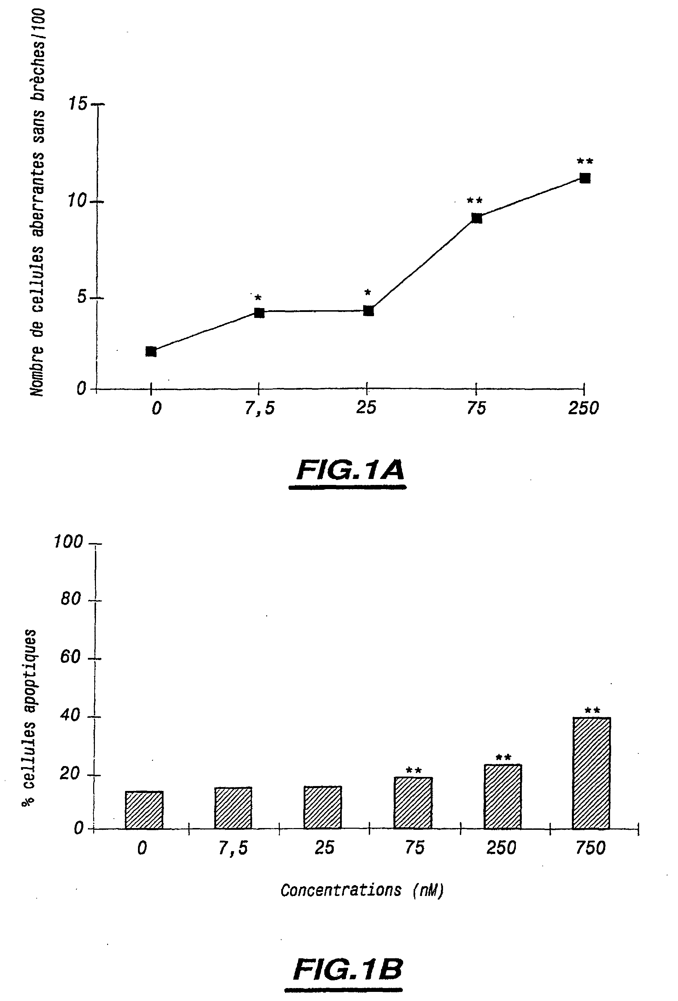

Dans l'analyse des métaphases *in vitro* sur des lymphocytes humains, le dexaméthasone (figures 1A et 1B) peut être considéré comme un agent clastogène, alors que ce composé est connu pour être un inducteur d'apoptose sans être génotoxique.

Les lymphocytes ont été traités pendant 44 heures avec une série de doses de 7,5 µM à 750 µM de dexaméthasone qui ont induit des aberrations de manière dose dépendante. A partir de 250 µM de dexaméthasone, l'apopotose, qui était d'environ 16 % dans le contrôle, a augmenté jusqu'à 40 % des cellules. 11 % de cellules aberrantes ont été observées contre 2 % dans le contrôle.

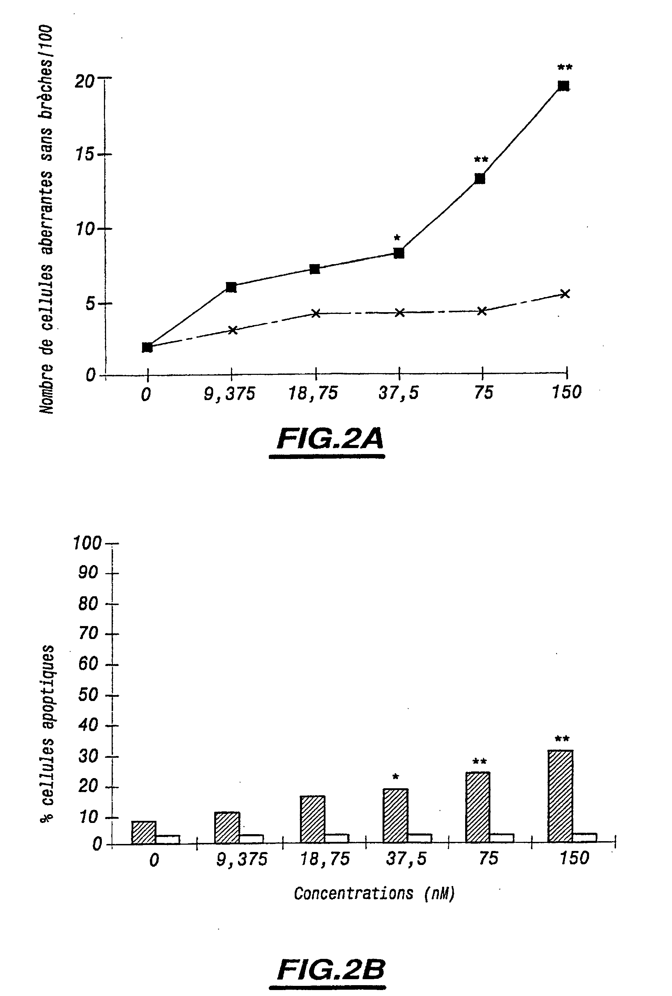

Afin de distinguer la part de l'apoptose dans les cassures des chromosomes et des chromatides, les auteurs de l'invention ont comparé les résultats obtenus dans des cellules CTLL-2 transfectées avec l'inhibiteur d'apoptose bcl2 par rapport aux résultats obtenus sur des cellules CTLL-2 non transfectées, dans une analyse de métaphases *in vitro*, avec une dose entre 9,575 nM et 150 nM de dexaméthasone (figures 2A et 2B). En parallèle, l'induction apoptotique a été mesurée. Dans les cellules CTLL-2, le nombre de cellules aberrantes était statistiquement différent (p=0,05) du contrôle, à partir de 37,5 nM de dexaméthasone. Il a en effet été observé 8 % de cellules aberrantes contre 2 % dans le contrôle.

Comme dans les lymphocytes humains, les auteurs de l'invention ont observé des cassures de chromosomes et de chromatides mais pas d'échange et dans le même temps, à partir de 37,5 nM de déxaméthasone, l'apopotose qui était d'environ 7 % dans le contrôle a augmenté jusqu'à 18 % atteignant 30 % à 150 nM. Ceci démontre la fonction apoptotique dans la fragmentation de l'ADN dans l'analyse des métaphases.

Le traitement de deux lignées cellulaires en analyse de métaphases avec 9,375 µM à 150 µM de méthional laisse apparaître des aberrations de structures comme des cassures ou des trous à partir de la seconde dose seulement dans les cellules non transfectées. La différence était statistiquement significative en comparaison avec le contrôle (p=0,05) avec 13 % de cellules porteuses d'aberrations chromosomiques à 18,75 µM (contre 3 % dans le contrôle).

L'analyse des métaphases des cellules traitées à partir de 25 jusqu'à 100 µM de gliotoxine a montré une réponse positive dans les cellules CTLL-2 à partir de 50 µM de gliotoxine en termes de clastogénèse (10 % de cellules aberrantes contre 1 % *chez les* témoins) mais on note également une forte augmentation de l'apoptose (17 % contre 5 % chez les témoins). A plus forte dose, les métaphases n'étaient plus analysables tandis que l'apoptose atteignait son maximum. Les résultats et les différences observés dans les cellules contenant l'inhibiteur d'apoptose contre ceux qui n'étaient pas transfectés montrent que le phénomène apoptotique conduit à des résultats de "faux-positifs" en termes de génotoxicité comme dans le test du micronoyau.

L'analyse des métaphases *in vitro* des lymphocytes humains traités par l'étoposide laisse apparaître une augmentation statistiquement significative du nombre de cellules aberrantes à partir de 125 nM jusqu'à 500 nM du composé, à de nombreux échanges chromosomiques et de réarrangements complexes, preuve d'une tentative de réparation de l'ADN.

Au delà de cette concentration, il n'est pas possible d'interpréter les métaphases car il y a trop de cassures et de réarrangements, et l'apoptose apparaît à partir de 2 000 nM d'étoposide pour atteindre 47 % (alors qu'il était d'environ 19 % dans le contrôle).

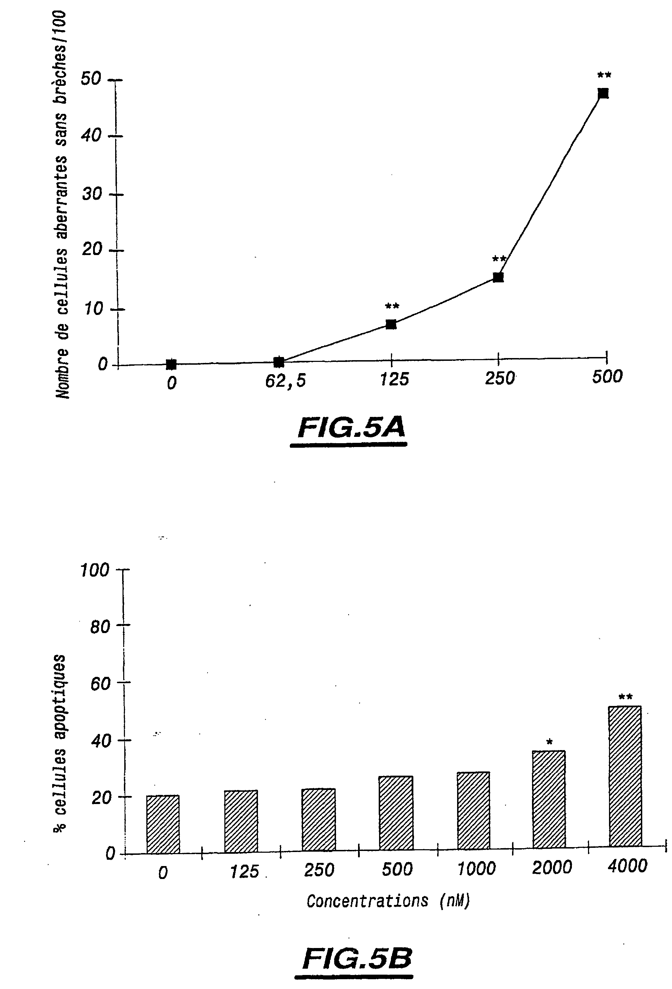

Ce test permet de qualifier l'étoposide comme étant un composé génotoxique (figures 5A et 5B).

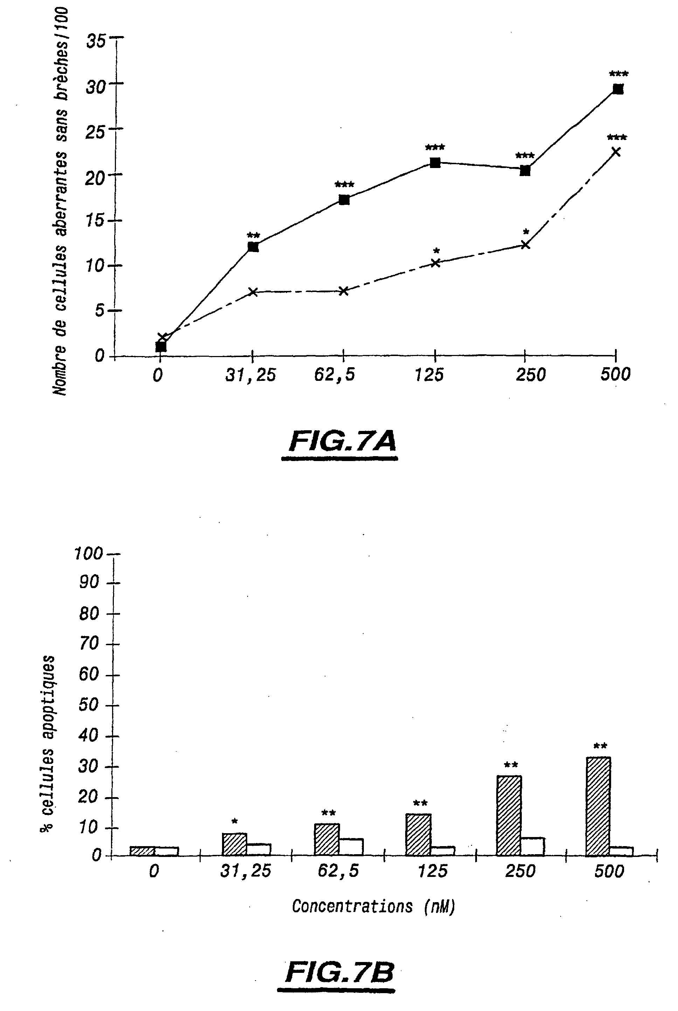

Dans le test du micronoyau, les auteurs de l'invention ont obtenu avec de fortes concentrations en étoposide respectivement 4,5 %₀ (contre 2,5 %₀ dans le contrôle) et 29 %₀ (contre 5 %₀ dans le contrôle) de cellules micronucléées avec l'inhibition complète de l'apoptose. Cette observation est également valable dans l'analyse de métaphases de cellules CTLL-2 traitées avec 31,25 à 500 nM d'étoposide (figures 7A et 7B). L'expérience révèle une augmentation statistiquement significative (p=0,01) dans le nombre de cellules aberrantes à partir de 62,5 nM jusqu'à 500 nM du composé, avec des échanges chromosomiques et des réarrangements complexes, preuve d'une tentative de réparation de l'ADN et l'apoptose augmentait également significativement à partir de la première dose d'étoposide. En retour, dans les cellules CTLL-2-bcl2 la différence (p=0,05) avec le contrôle est apparue à partir de 125 nM du composé, sans apoptose.

### F - Conclusion

Comme dans le test du micronoyau, ces derniers résultats permettent de montrer que l'apoptose peut engendrer des résultats faussement positifs également dans le test d'analyses de métaphases dans les cellules CTLL-2 alors que la transfection par bcl2 éradique ces événements.

Grâce à ces résultats en accord avec ceux qui ont été observés dans le test *in vitro* du micronoyau dans le modèle CTLL-2/ CTLL-2-bcl2, on dispose donc d'un nouveau test : le test d'analyses de métaphases *in vitro* qui décèle les aberrations de structure chromosomiques ou chromatidiques et les aberrations numériques.

### REFERENCES BIBLIOGRAPHIQUES

- Biolo et al, (1999), Immunoanal. Biol. Spéc, n° 14, 16-31 Editions Elsevier, Paris
- Bolse et al, (1993), Cell, vol. 74, 597-608
- Chao et al, (1995), J. Exp. Med., vol. 182, 821-828
- Deng et al, (1993), Proc. Natl. Acad. Sci. USA, vol. 90, 2189-2193
- Evans et al, (1987), dans Handbook of Mutagenicity test procedure, second Edition, Kilbey B.J. et al, Elsevier, 405-428
- Gillis et al, (1977), J. exp. Med., n° 14, 468-482
- Hu et al, (1997), Cancer Res., n° 57, 3339-3343
- Kirch-Volders et al, (1997), Mutat. Res., n° 392, 19-30
- Kirkland et al, (1989), Mutat. Res., n° 214, 115-122
- Matsuoka et al, (1993) Mutat. Res., n° 272, 223-236
- Renvoizé et al, (1997), The Journal of Immunology, 159:126-134

## Revendications

1. Procédé d'évaluation *in vitro* de la génotoxicité d'un composé, dans lequel on met en contact ledit composé avec au moins une cellule surexprimant le protooncogène bcl2 et/ou une protéine anti-apoptotique apparentée, et on observe les effets génotoxiques dudit composé sur ladite cellule.

2. Procédé selon la revendication 1, dans lequel on observe les effets génotoxiques positifs du composé par la formation de micronoyau(x).

3. Procédé selon la revendication 1, dans lequel on observe les effets génotoxiques positifs du composé par la présence d'anomalies de nombre et/ou de structure des chromosomes en métaphase.

4. Procédé selon l'une quelconque des revendications précédentes, dans lequel la cellule surexprime bcl2.

5. Procédé selon l'une quelconque des revendications précédentes, dans lequel la cellule surexprime bcl-XL.

6. Procédé selon l'une quelconque des revendications précédentes, dans lequel la cellule est une cellule de la lignée murine CTLL-2 transfectée.

7. Procédé selon l'une quelconque des revendications précédentes, dans lequel ledit composé à tester est sous la forme d'un mélange de plusieurs molécules.

8. Procédé selon l'une quelconque des revendications précédentes, dans lequel on ajoute audit composé à tester un activateur métabolique de celui-ci.

9. Utilisation de cellules de mammifères surexprimant le protooncogène bcl2 pour évaluer la génotoxicité d'un composé.

## Patentansprüche

1. Verfahren zur *in vitro*-Beurteilung der Genotoxizität einer Verbindung, bei dem die besagte Verbindung mit wenigstens einer das Protoonkogen bcl2 und/oder ein verwandtes antiapoptotisches Protein überexprimierender Zelle in Kontakt gebracht wird und die genotoxischen Wirkungen der besagten Verbindung auf die besagte Zelle beobachtet werden.

2. Verfahren nach Anspruch 1, bei dem die positiven genotoxischen Wirkungen der Verbindung durch die Bildung eines Mikrokerns bzw. von Mikrokernen beobachtet wird.

3. Verfahren nach Anspruch 1, bei dem die positiven genotoxischen Wirkungen der Verbindung durch das Vorhandensein von zahlenmäßigen und/oder strukturellen Anomalien der Chromosomen in der Metaphase beobachtet werden.

4. Verfahren nach einem der vorangehenden Ansprüche, bei dem die Zelle bcl2 überexprimiert.

5. Verfahren nach einem der vorangehenden Ansprüche, bei dem die Zelle bcl-XL überexprimiert.

6. Verfahren nach einem der vorangehenden Ansprüche, bei dem die Zelle eine transfektierte Zelle der Mäusezellinie CTLL-2 ist.

7. Verfahren nach einem der vorangehenden Ansprüche, bei dem die besagte zu testende Verbindung in Form einer Mischung von mehreren Molekülen vorliegt.

8. Verfahren nach einem der vorangehenden Ansprüche, bei dem der besagten zu testenden Verbindung ein Stoffwechselaktivator für diese Verbindung zugesetzt wird.

9. Verwendung von das Protoonkogen bcl2 überexprimierenden Säugetierzellen zur Beurteilung der Genotoxizität einer Verbindung.

## Claims

1. Method for assessing the genotoxicity of a compound, *in vitro,* in which the said compound is brought into contact with at least one cell overexpressing the bcl2 proto-oncogene and/or a related anti-apoptotic protein, and the genotoxic effects of the said compound on the said cell are observed.

2. Method according to Claim 1, in which the positive genotoxic effects of the compound are observed by the formation of a micronucleus or micronuclei.

3. Method according to Claim 1, in which the positive genotoxic effects of the compound are observed by the presence of abnormalities of number and/or of structure of the chromosomes in metaphase.

4. Method according to any one of the preceding claims, in which the cell overexpresses bcl2.

5. Method according to any one of the preceding claims, in which the cell overexpresses bcl-XL.

6. Method according to any one of the preceding claims, in which the cell is a cell of the CTLL-2 murine line which has been transfected.

7. Method according to any one of the preceding claims, in which the said compound to be tested is in the form of a mixture of several molecules.

8. Method according to any one of the preceding claims, in which a metabolic activator of the said compound to be tested is added thereto.

9. Use of mammalian cells overexpressing the bcl2 proto-oncogene, for assessing the genotoxicity of a compound.
